# EUROPEAN PATENT APPLICATION

(11) **EP 4 606 334 A1**
(43) Date of publication of application: **27.08.2025**
(21) Application number: 25159255.6
(22) Date of filing: 21.02.2025
(51) Int. Cl.: A61B 18/14, A61B 17/00, A61B 18/00

(54) **MODULAR RING ELECTRODES**

(30) Priority: 22.02.2024 US 202418584855
(71) Applicant: Biosense Webster (Israel) Ltd., 2066717 Yokneam (IL)
(72) Inventor: ROUSU, Corey M., Irvine, 92618 (US)
(74) Representative: Carpmaels & Ransford LLP

(57) **Abstract**

The disclosed technology includes an end effector for a catheter. The end effector comprises a plurality of ring electrode assemblies, each ring electrode comprising a ring electrode disposed around an insert. Collars can be attached to each end of the ring electrode to fix the ring electrode to the insert. Multiple ring electrode assemblies can be coupled to one another with bridge tubes to form a length of an end effector.

## Description

### FIELD

The present technology relates generally to minimally invasive medical devices, and in particular cardiac mapping catheters with flexible end effectors.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors or electrodes into the heart and acquiring data at multiple points. These data are then utilized to select the target areas at which ablation is to be performed.

For prevention of unwanted damage to regions surrounding the target anatomy, it may be desirable to provide an end effector with as small as footprint as possible. It may also be desirable to provide a plurality of electrodes on the end effector to collect larger amounts of data signals and/or make the most use of the available surface area provided by the end effector. However, assembly of such end effectors with small components can prove to be a tedious and difficult task. Accordingly, there is a need for an improved electrode assembly that can facilitate manufacturing of the end effector of a catheter.

### SUMMARY

There is provided, in accordance with the disclosed technology, a ring electrode assembly including an insert comprising at least one lumen extending along a longitudinal axis, a ring electrode disposed around a middle portion of the insert and having two ends, and two collars, each collar coupled to each of the two ends of the ring electrode and to the insert, thereby fixing the ring electrode to the insert.

The ring electrode can taper inward from a center portion toward each of the two ends. The inner circumference of the two ends of the ring electrode can be approximately equal to an outer circumference of the middle portion of the insert. Each of the collars may taper inward from a first end toward a second end, and an inner circumference of the first end of each collar can be approximately equal to an outer circumference of the two ends of the ring electrode. Each end of the insert can comprise a first ledge, the first ledge having an outer circumference approximately equal to an inner circumference of the second end of each collar. Each end of the insert can further comprise a second ledge, the first ledge being provided between the second ledge and the middle portion of the insert at each end of the insert, and the outer circumference of the middle portion of the insert can be greater than the outer circumference of the first ledge, and the outer circumference of the first ledge can be greater than an outer circumference of the second ledge. A length of the second ledge can be approximately 0.5 mm. The ring electrode can include a plurality of irrigation holes. The inner circumference of the center portion of the ring electrode can greater than an outer circumference of the middle portion of the insert, and a resulting gap between the inner circumference of the center portion of the ring electrode and the outer circumference of the middle portion of the insert can provide an irrigation reservoir.

There is provided, in accordance with the disclosed technology, an end effector of a catheter including two or more ring electrode assemblies, where each ring electrode assembly can include an insert having at least one lumen extending along a longitudinal axis, the insert further comprising a middle portion, a second ledge provided at each end of the insert, and a first ledge provided between each second ledge and the middle portion, a ring electrode can be provided, the ring electrode having two ends and disposed around the middle portion of the insert. The ring assembly can further include two collars, each collar coupled to each of the two ends of the ring electrode. The end effector can also include least one bridge tube coupling adjacent ring electrode assemblies. The bridge tube can include at least one lumen corresponding to the at least one lumen of the insert. The end effector can further include a frame provided through the at least one lumen of each ring electrode assembly and through the at least one lumen of the at least one bridge tube.

The frame can comprise nitinol. The at least one bridge tube can include two ends, and the two ends of the at least one bridge tube can have an inner diameter which is approximately equal to an outer diameter of the second ledge. The ring electrode can taper inward from its center toward each of its two ends. An inner diameter of each of the two ends of the ring electrode can be approximately equal to an outer diameter of the middle portion of the insert. Each of the collars can taper inward from a first end toward a second end, and an inner diameter of the first end of each collar can approximately equal to an outer diameter of the two ends of the ring electrode. An inner diameter of the second end of each collar can be approximately equal to an outer diameter of the first ledge. The end effector can further comprise an overmold between each collar and each first ledge of the insert. At least one lumen of the insert can comprise a lead wire lumen. A lead wire hole can extend from the lead wire lumen to an outer surface of the insert.

There is provided, in accordance with the disclosed technology, a method for assembling an end effector of a catheter comprising assembling one or more ring electrode assemblies, where assembling each ring electrode assembly comprises providing an insert, sliding a ring electrode onto the middle portion of the insert, and sliding a collar onto each end of the ring electrode. Each collar can slide onto a respective first ledge of the insert. Each insert can include at least one lumen extending along a longitudinal axis, a middle portion, a second ledge provided at each end of the insert, and a first ledge provided between each second ledge and the middle portion.

Assembling the end effector can further include sliding each ring electrode assembly over a frame, such that the frame is provided through the at least one lumen of the insert. The method can further include assembling at least two ring electrode assemblies and coupling adjacent ring electrodes assemblies together with a bridge tube. coupling the bridge tube to each ring assembly can include sliding an end of the bridge tube over the second ledge of each insert. Each end of the bridge tube can comprise an inner circumference approximately equal to an outer circumference of the second ledge.

### BRIEF DESCRIPTION OF THE DRAWINGS

FIG. 1 is a schematic pictorial illustration of a medical system including a catheter comprising a plurality of ring electrodes, in accordance with the disclosed technology;
FIG. 2A is a schematic pictorial illustration of an insert component of a modular ring electrode assembly, in accordance with the disclosed technology;
FIG. 2B is an end view of the insert component shown in FIG. 2A of a modular ring electrode assembly, in accordance with the disclosed technology;
FIG. 3 is a schematic pictorial illustration of a ring electrode component of a modular ring electrode assembly, in accordance with the disclosed technology;
FIG. 4 is a schematic pictorial illustration of a ring electrode component disposed over an insert component of a modular ring electrode assembly, in accordance with the disclosed technology;
FIG. 5A is a schematic pictorial illustration of a modular ring electrode assembly, in accordance with the disclosed technology;
FIG. 5B is a cross-sectional view of the modular ring electrode assembly taken along line A-A of FIG. 5A, in accordance with the disclosed technology;
FIG. 6 is a schematic pictorial illustration of a modular ring electrode assembly, in accordance with the disclosed technology; and
FIG. 7 is a flow diagram depicted a method of assembling a modular ring electrode assembly, in accordance with the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the present disclosure. The detailed description illustrates by way of example, not by way of limitation, the principles of the disclosed technology. This description will clearly enable one skilled in the art to make and use the disclosed technology, and describes several embodiments, adaptations, variations, alternatives and uses of the disclosed technology, including what is presently believed to be the best mode of carrying out the disclosed technology.

As used herein, the terms "about" or "approximately" or "generally" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject technology in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular", "tube" and "shaft" are to be construed broadly and are not limited to a structure that is a right cylinder or strictly circumferential in cross-section or of a uniform cross-section throughout its length. For example, the tubular/shaft structures are generally illustrated as a substantially right cylindrical structure. However, the tubular/shaft structures may have a tapered or curved outer surface without departing from the scope of the present disclosure.

The present disclosure is related to systems, method or uses and devices for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for irreversible electroporation (IRE), RF ablation, and/or cryoablation. IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The technology of this disclosure includes systems and methods for applying electrical signals from catheter electrodes positioned in the vicinity of myocardial tissue to generate a generate ablative energy to ablate the myocardial tissue. In some examples, the systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210, the entirety of which is incorporated herein by reference.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. Examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1, the entireties of each of which are incorporated herein by reference.

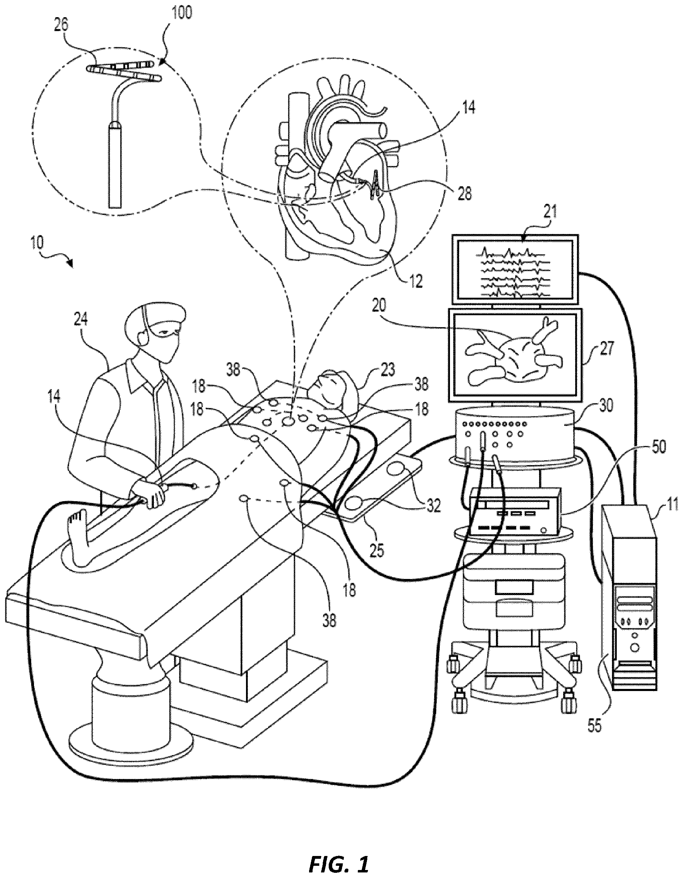

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft 90 with distal tip of catheter 14 (i.e., end effector 100) into contact with the heart wall for sensing a target site in heart 12. For ablation, physician 24 would similarly bring a distal end of an ablation catheter to a target site for ablating.

Catheter 14 is an exemplary catheter that includes one and preferably multiple electrodes 26 optionally distributed over end effector 100 coupled to a catheter shaft 90 and configured to sense the IEGM signals as described in more detail below. Catheter 14 may additionally include a position sensor embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip 28 of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

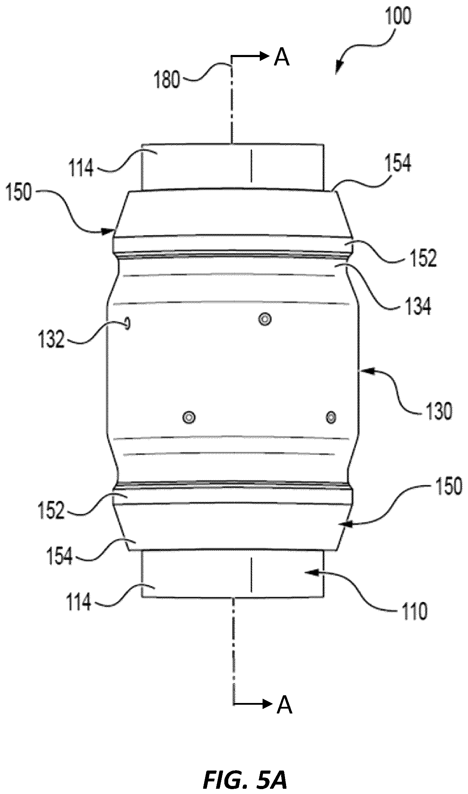

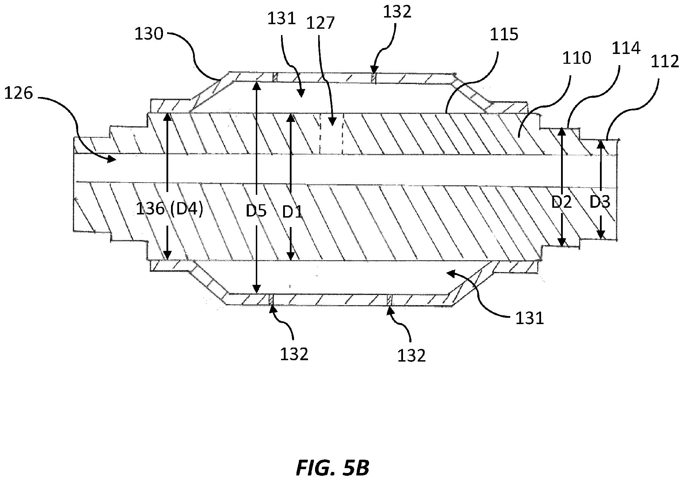

FIG. 5 depicts a ring electrode assembly 100, in accordance with some examples of the disclosed technology. The ring electrode assembly can comprise an insert 110, a ring electrode 130 disposed over the insert 110, and two collars 150 coupled to the ends of the ring electrode 130 and the insert 110 to fix the ring electrode to the insert 110.

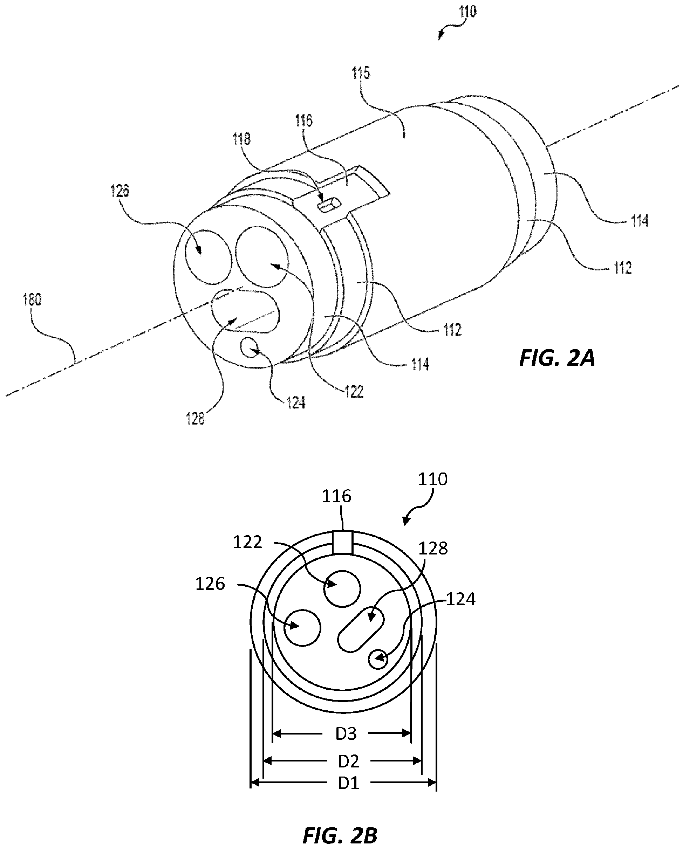

FIGs. 2A and 2B depict the insert component 110 of a modular ring assembly 100, in accordance with some examples of the disclosed technology. FIG 2A is a perspective while FIG. 2B is an end view of the insert component 110. The insert 110 can extend along a longitudinal axis 180 and include a middle portion 115 having a first diameter or circumference D1. A first ledge 112 can be provided at each end of the insert 110, having a second diameter D2 or second circumference which is slightly less than the first diameter or first circumference of the middle portion 115 of the insert 110. A second ledge 114 can be provided at each end of the insert 110, having a third diameter D3 or third circumference which is slightly less than the second diameter or second circumference of the first ledge 112. The arrangement is such that the ends of the insert 110 can comprise a two-step taper provided by first ledges 112 and the second ledges 114. The second ledge 114 is provided at the ends of the insert 110, and first ledges 112 are provided between the second ledges 114 and the middle portion 115 of the insert 110. In some examples, the first ledges 112 comprises a length of about 0.5 millimeters (mm). In some examples, the second ledges 114 comprises a length of about 0.5 millimeters (mm).

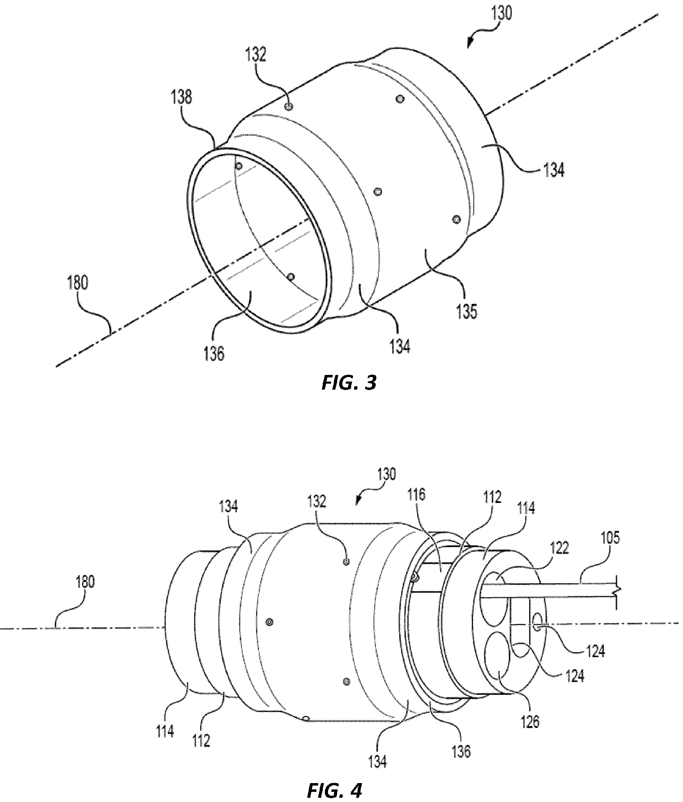

The insert 110 can further comprise one or more lumens 122, 124, 126, 128. In some examples, the insert 110 comprises a lead wire lumen 122 for providing a lead wire (e.g., lead wire 105 depicted in FIG. 4) for providing an electrical signal to the ring electrodes of the end effector. The insert 110 can further include a lead wire hole 118 for providing a lead wire to a ring electrode when the ring electrode is disposed around the insert (as depicted in FIG. 4). The insert 110 can further comprise a lead wire recess 116 providing a space between the insert 110 and the ring electrode for the lead wire.

The insert 110 can further comprise a pull wire lumen 124. In some examples, the end effector is a helical end effector (as depicted in FIG. 1). The helical end effector can change its shape, for example, from straightened configuration to the helical configuration (as depicted in FIG. 1) by engaging a pull wire which can be provided through the pull wire lumen 124.

The insert 110 can further comprise an irrigation lumen 126. In some examples, irrigation fluid is provided through irrigation tubes which can be disposed within the irrigation lumen 126. In some examples, the irrigation lumen 126 acts as an irrigation tube, providing a channel for irrigation fluid to flow through.

The insert 110 can further comprise a frame lumen 128. The frame lumen 128 can be provided to receive a frame which forms the shape of the end effector. In some examples, the frame is formed from a flexible, resilient material. By way of example, the frame can be formed from a shape-memory alloy such as nickel-titanium, also known as Nitinol, cobalt chromium, stainless steel, and/or other alloys that exhibit pseudoelastic properties. In some examples, the frame lumen 128 is shaped to correspond to a cross-section of the frame. For example, the frame lumen 128 can comprise an elongated slot corresponding to an ovoid cross section of a frame.

The insert can comprise a single integral piece. The insert can be molded. The insert can be formed by injection molding. In some examples, the insert comprises a plastic or an elastomeric material. The insert can also comprise an insulative material or coated with an insulator.

FIG. 3 depicts a ring electrode 130 of the modular ring electrode assembly, according to some examples. The ring electrode 130 can comprise an annular body extending along a longitudinal axis 180. The ring electrode 135 can comprise one or more irrigation holes 132 for delivering irrigation fluid to a target anatomical region. The irrigation fluid can be used to facilitate cooling of the target anatomical region and the ring electrode 130 during operation of the catheter during, for example, ablative procedures. The ring electrode 130 further comprises two tapered ends 134. A taper is provided at the ends of the ring electrode 130 such that at least the outer diameter/circumference of the middle portion 135 of the ring electrode 130 is larger than the outer diameters/circumferences 138 of the tapered ends 134.

FIG. 4 depicts the ring electrode 130 disposed over an insert 110, according to some examples. As shown in FIG. 5B, which is a cross-sectional view of the ring electrode 130 and the insert 110 taken along line A-A of FIG. 5A, the inner diameter/circumference 136 (D4) of the tapered ends 134 of the electrode can be approximately equal to the outer diameter/circumference D1 of the middle portion 115 of the insert 110. Disposal of the ring electrode 130 onto the insert 110 can create an interference fit between the inner diameter/circumference 136 of the tapered ends 134 and the outer diameter/circumference D1 of the middle portion 115 of the insert 110 such that a seal is created at the interface between the tapered ends 134 of the ring electrode 130 and the outer circumference of the middle portion 115 of the insert 110. In some examples, the middle portion of the ring electrode 130 comprises an inner diameter/circumference D5 greater than the outer diameter/circumference (D1) of the middle portion of the insert 110, thereby providing a space 131 between middle portion of the insert 110 and the middle portion of the ring electrode 130. This provided space 131 can be utilized a reservoir for the introduction of irrigation fluid. In other examples, the lead wire hole (hole 118 depicted in FIG. 2) can also be used to accommodate an irrigation fluid tube and introduce irrigation fluid into the space 131 provided between middle portion of the insert 110 and the middle portion of the ring electrode 130. In some examples, one or more additional holes 127 are provided through the middle portion 115 of the insert 110 from the irrigation fluid lumen 126 to the outer circumference/surface of the middle portion 115 of the insert 110 for providing an irrigation tube or irrigation fluid to an irrigation reservoir provided by a space 131 between middle portion 115 of the insert 110 and the middle portion of the ring electrode 130.

Referring again to FIG. 5A, an exemplary modular ring electrode assembly 100 is depicted with the ring electrode 130 disposed over the insert 110 and collars 150 coupled to each end of the ring electrode 130, thereby fixing the ring electrode 130 to the insert 110. The collars 150 can be connected to the ring electrode 130 and/or the insert 110 by a press fit, by an adhesive, shrink tubing, crimping, or other suitable methods of attaching the collar to the ring electrode 130 and/or the insert 110. In some examples, each of the collars 150 taper from a first end 152 to a second end 154. The first end 152 of each collar 150 can comprise an inner diameter/circumference which approximately equal to an outer diameter/circumference of the tapered ends 134 of the ring electrode 130. The second end 154 of each collar 150 can comprise an inner diameter/circumference which is approximately equal to an outer diameter/circumference of the first ledge 112 of the insert 110. In some examples, when the collars 150 are coupled to the tapered ends 134 of the ring electrode 130 and the first ledge 112 of the insert 110, the ring electrode 130 becomes fixed in position relative to the insert 110. The collars 150 may also provide a seal for preventing a fluid from passing between the tapered ends 134 of ring electrode 130 and the insert 110. In some examples, after coupling of the collars 150 to the ends 134 of the ring electrode 134, the second ledges 114 of the insert 110 protrude outward from the collars 150. In some examples, an overmold is provided over or into the collars 150 to fix the collars to the ring electrode 130, which may also fix the ring electrode to the insert 110. In some examples, the outer diameter of the insert 110 is roughened or provided with a surface roughness to facilitate adhesion of the overmold. In some examples, a space is provided between an inner diameter of the collars 150 and the first ledge 112 to be filled in with the overmold material. The collars 150 may comprise slots or through holes for injection of overmold material. In some examples, mandrels are placed in the lumens of the inserts during injection of overmold material. The collars 150 can also be swaged onto the electrode 130 and/or the insert 110.

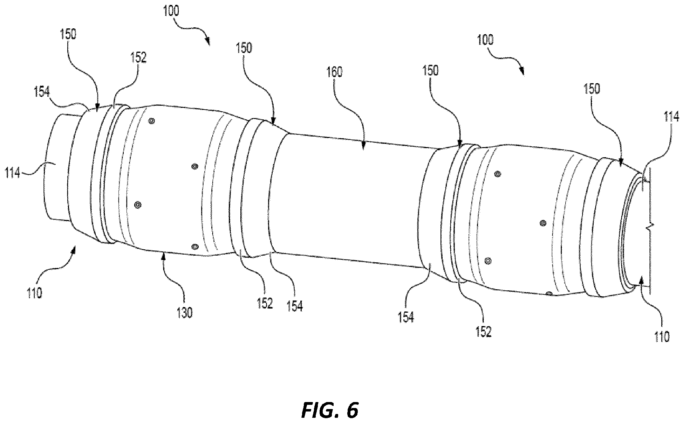

As depicted by FIG. 6, in some examples, two or more ring electrode assemblies 100 can be connected by one or more bridge tubes 160. In some examples, a bridge tube 160 is provided between two adjacent ring electrode assemblies 100. The ends of the bridge tube 160 can comprise an inner diameter/circumference approximately equal to the outer diameter/circumference of the second ledge 114 of the insert 100. In some examples, a bridge tube 160 can be fitted onto the second ledges 114 of two adjacent ring electrode assemblies, thereby connecting the two adjacent ring electrode assemblies 100. A plurality of ring electrode assemblies can be connected by multiple bridge tubes 160 to provide an end effector of a desired length.

In some examples, a bridge tube 160 can comprise a plurality of lumens which correspond to the lumens of the inserts 110. For example, a bridge tube 160 can comprise four lumens which correspond to the lead wire lumen 122, a pull wire lumen 124, an irrigation lumen 126, and a frame lumen 128 of the insert 110 depicted in FIG. 2. In some examples, the lumens of the bridge tube 160 are aligned with the lumens of the insert 110. In some examples, the bridge tube 160 can comprise a single lumen to accommodate the components (e.g., a pull wire, frame, irrigation tube, and/or lead wire) of the end effector. In some examples, after the bridge tube 160 is fitted and adhered onto the second ledges 114 of the inserts 110 to fix the bridge tube 160 to the ring assemblies 100. A suitable adhesive may include polyurethane. In some examples, mandrels are utilized to prevent occlusion of the lumens of the bridge tubes 160 and/or inserts 110 during application of the adhesive. In some examples, through-holes may be provided in a bridge tube 110, via trepanning or another suitable method, to facilitate application of an adhesive.

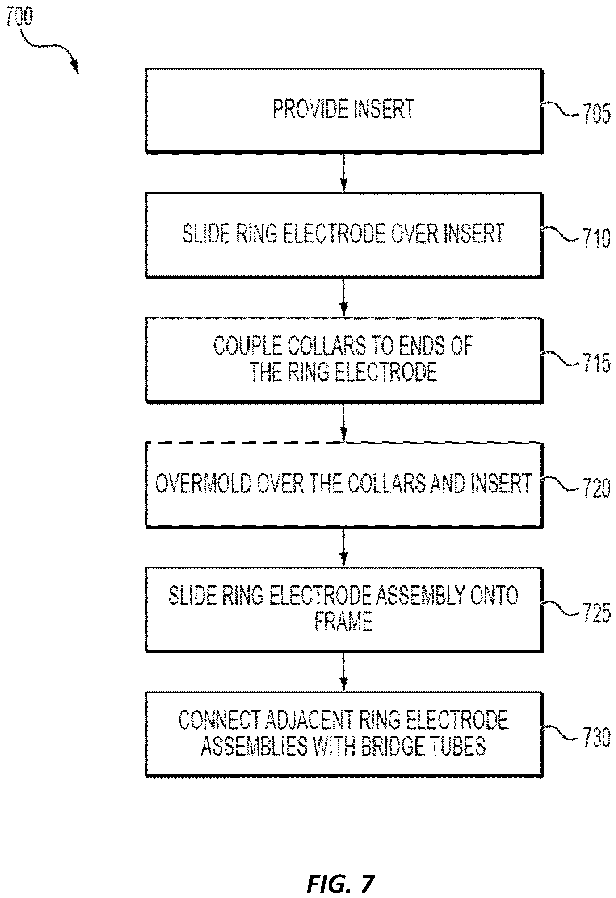

FIG. 7 depicts an exemplary method 700 for forming an end effector using the modular ring assemblies disclosed herein. In some examples, the method 700 begins by providing an insert (e.g., insert 110 depicted in FIG. 2) at a first step 705. At a second step 710, a ring electrode can be slide over the insert (as depicted in FIG. 4). As described herein, the ring electrode can be sized such that an interference fit is provided between the insert and ring electrode to temporarily hold the ring electrode in place. At a third step 715, collars may be coupled to the ends of the ring electrode. In some examples, the collars are also coupled to the ends of the inserts during coupling to ends (e.g., the first ledges 112) of the ring electrode. Coupling of the collars to the ends of ring electrode and ends of the insert can fix the ring electrode to the insert. At a fourth step 720, an overmold can be provided over or into the collars and the insert to fix the ring electrode and the collars to the insert. At a fifth step 725, the ring electrode assembly (e.g., ring electrode assembly 100 depicted in FIG. 5), is slide over a frame of the end effector. As an optional sixth step 730, a bridge tube (e.g., bridge tube 160 depicted in FIG. 6) is slide over the frame and an additional ring electrode assembly can then be slide over the frame at the other end of the bridge tube, thereby connecting the ring electrode assemblies. Multiple ring electrode assemblies can be connected by bridge tubes to form an end effector of a desired length. Optionally, and in addition to or in lieu of the overmolding of the fourth step 720, an overmold can be provided over or into the collars and extending onto the ends of the bridge tubes to secure the ring electrode assemblies to the bridge tubes.

The disclosed technology described herein can be further understood according to the following clauses:
Clause 1. A ring electrode assembly comprising: an insert comprising at least one lumen extending along a longitudinal axis; a ring electrode disposed around a middle portion of the insert and having two ends; and two collars, each collar coupled to each of the two ends of the ring electrode and to the insert, fixing the ring electrode to the insert.
Clause 2. The ring electrode assembly of Clause 1, the ring electrode is tapered inward from a center portion toward each of the two ends.
Clause 3. The ring electrode assembly of Clause 2, an inner circumference of each of the two ends of the ring electrode is approximately equal to an outer circumference of the middle portion of the insert.
Clause 4. The ring electrode assembly of Clause 2 or 3, each of the two collars tapers inward from a first end toward a second end, and an inner circumference of the first end of each collar is approximately equal to an outer circumference of the two ends of the ring electrode.
Clause 5. The ring electrode assembly of Clause 4, each end of the insert comprises a first ledge, the first ledge having an outer circumference approximately equal to an inner circumference of the second end of each collar.
Clause 6. The ring electrode assembly of Clause 5, each end of the insert further comprises a second ledge, the first ledge is provided between the second ledge and the middle portion of the insert at each end of the insert, and the outer circumference of the middle portion of the insert is greater than the outer circumference of the first ledge, and the outer circumference of the first ledge is greater than an outer circumference of the second ledge.
Clause 7. The ring electrode of Clause 6, a length of the second ledge is approximately 0.5 mm.
Clause 8. The ring electrode of any one of Clauses 2 to 7, the ring electrode comprises a plurality of irrigation holes, and an inner circumference of the center portion of the ring electrode is greater than an outer circumference of the middle portion of the insert, a resulting gap between the inner circumference of the center portion of the ring electrode and the outer circumference of the middle portion of the insert provides an irrigation reservoir.
Clause 9. An end effector of a catheter comprising: two or more ring electrode assemblies, each ring assembly comprising: an insert comprising at least one lumen extending along a longitudinal axis, the insert further comprising a middle portion, a second ledge provided at each end of the insert, and a first ledge provided between each second ledge and the middle portion; a ring electrode disposed around the middle portion of the insert and having two ends; and two collars, each collar coupled to each of the two ends of the ring electrode; at least one bridge tube coupling adjacent ring electrode assemblies and comprising at least one lumen corresponding to the at least one lumen of the insert; and a frame provided through the at least one lumen of each ring electrode assembly and through the at least one lumen of the at least one bridge tube.
Clause 10. The end effector of Clause 9, the frame comprises nitinol.
Clause 11. The end effector of Clause 9 or 10, the at least one bridge tube comprises two ends, the two ends of the at least one bridge tube having an inner diameter being approximately equal to an outer diameter of the second ledge.
Clause 12. The end effector of any one of Clauses 9 to 11, the ring electrode is tapered inward from a center portion toward each of the two ends, and an inner diameter of each of the two ends is approximately equal to an outer diameter of the middle portion of the insert.
Clause 13. The end effector of any one of Clauses 9 to 12, the each of the two collars tapers inward from a first end toward a second end, and an inner diameter of the first end of each collar is approximately equal to an outer diameter of the two ends of the ring electrode.
Clause 14. The end effector of Clause 13, an inner diameter of the second end of each collar is approximately equal to an outer diameter of the first ledge.
Clause 15. The end effector of Clauses 9 to 14, further comprising an overmold between each collar and each first ledge of the insert.
Clause 16. The end effector of any one of Clauses 9 to 15, the at least one lumen of the insert comprises a lead wire lumen, and the insert further comprises a lead wire hole extending from the lead wire lumen to an outer surface of the insert.
Clause 17. A method for assembling an end effector of a catheter comprising: assembling one or more ring electrode assemblies, assembling each ring electrode assembly comprises: providing an insert comprising at least one lumen extending along a longitudinal axis, the insert further comprising a middle portion, a second ledge provided at each end of the insert, and a first ledge provided between each second ledge and the middle portion; sliding a ring electrode onto the middle portion of the insert; and sliding a collar onto each end of the ring electrode, each collar sliding onto a respective first ledge of the insert.
Clause 18. The method of Clause 17, assembling the end effector further comprises sliding each ring electrode assembly over a frame, such that the frame is provided through the at least one lumen of the insert.
Clause 19. The method of Clause 17 or 18, at least two ring electrode assemblies are assembled, and the method further comprises coupling adjacent ring electrodes assemblies together with a bridge tube.
Clause 20. The method of Clause 19, coupling the bridge tube to each ring assembly comprises sliding an end of the bridge tube over the second ledge of each insert, each end of the bridge tube comprises an inner circumference approximately equal to an outer circumference of the second ledge.

The examples described above are cited by way of example, and the disclosed technology is not limited by what has been particularly shown and described hereinabove. Rather, the scope of the disclosed technology includes both combinations and sub combinations of the various features described hereinabove, as well as variations and modifications thereof which would occur to persons skilled in the art upon reading the foregoing description and which are not disclosed in the prior art.

## Claims

1. A ring electrode assembly comprising:
an insert comprising at least one lumen extending along a longitudinal axis;
a ring electrode disposed around a middle portion of the insert and having two ends; and
two collars, each collar coupled to each of the two ends of the ring electrode and to the insert, fixing the ring electrode to the insert.

2. The ring electrode assembly of claim 1, the ring electrode is tapered inward from a center portion toward each of the two ends, optionally wherein an inner circumference of each of the two ends of the ring electrode is approximately equal to an outer circumference of the middle portion of the insert.

3. The ring electrode assembly of claim 2, each of the two collars tapers inward from a first end toward a second end, and an inner circumference of the first end of each collar is approximately equal to an outer circumference of the two ends of the ring electrode.

4. The ring electrode assembly of claim 3, each end of the insert comprises a first ledge, the first ledge having an outer circumference approximately equal to an inner circumference of the second end of each collar, optionally wherein each end of the insert further comprises a second ledge, the first ledge is provided between the second ledge and the middle portion of the insert at each end of the insert, and the outer circumference of the middle portion of the insert is greater than the outer circumference of the first ledge, and the outer circumference of the first ledge is greater than an outer circumference of the second ledge, further optionally wherein a length of the second ledge is approximately 0.5 mm.

5. The ring electrode of any of claims 2 to 4, the ring electrode comprises a plurality of irrigation holes, and an inner circumference of the center portion of the ring electrode is greater than an outer circumference of the middle portion of the insert, a resulting gap between the inner circumference of the center portion of the ring electrode and the outer circumference of the middle portion of the insert provides an irrigation reservoir.

6. An end effector of a catheter comprising:
two or more ring electrode assemblies, each ring electrode assembly comprising:
an insert comprising at least one lumen extending along a longitudinal axis, the insert further comprising a middle portion, a second ledge provided at each end of the insert, and a first ledge provided between each second ledge and the middle portion;
a ring electrode disposed around the middle portion of the insert and having two ends; and
two collars, each collar coupled to each of the two ends of the ring electrode;
at least one bridge tube coupling adjacent ring electrode assemblies and comprising at least one lumen corresponding to the at least one lumen of the insert; and
a frame provided through the at least one lumen of each ring electrode assembly and through the at least one lumen of the at least one bridge tube.

7. The end effector of claim 6, the frame comprises nitinol.

8. The end effector of claim 6 or claim 7, the at least one bridge tube comprises two ends, the two ends of the at least one bridge tube having an inner diameter being approximately equal to an outer diameter of the second ledge.

9. The end effector of any of claims 6 to 8, the ring electrode is tapered inward from a center portion toward each of the two ends, and an inner diameter of each of the two ends is approximately equal to an outer diameter of the middle portion of the insert.

10. The end effector of any of claims 6 to 9, the each of the two collars tapers inward from a first end toward a second end, and an inner diameter of the first end of each collar is approximately equal to an outer diameter of the two ends of the ring electrode, optionally wherein an inner diameter of the second end of each collar is approximately equal to an outer diameter of the first ledge.

11. The end effector of any of claims 6 to 10, further comprising an overmold between each collar and each first ledge of the insert.

12. The end effector of any of claims 6 to 11, the at least one lumen of the insert comprises a lead wire lumen, and the insert further comprises a lead wire hole extending from the lead wire lumen to an outer surface of the insert.

13. A method for assembling an end effector of a catheter comprising:
assembling one or more ring electrode assemblies, assembling each ring electrode assembly comprises:
providing an insert comprising at least one lumen extending along a longitudinal axis, the insert further comprising a middle portion, a second ledge provided at each end of the insert, and a first ledge provided between each second ledge and the middle portion;
sliding a ring electrode onto the middle portion of the insert; and
sliding a collar onto each end of the ring electrode, each collar sliding onto a respective first ledge of the insert.

14. The method of claim 13, assembling the end effector further comprises sliding each ring electrode assembly over a frame, such that the frame is provided through the at least one lumen of the insert.

15. The method of claim 13 or claim 14, at least two ring electrode assemblies are assembled, and the method further comprises coupling adjacent ring electrodes assemblies together with a bridge tube, optionally wherein coupling the bridge tube to each ring assembly comprises sliding an end of the bridge tube over the second ledge of each insert, each end of the bridge tube comprises an inner circumference approximately equal to an outer circumference of the second ledge.
